# EUROPEAN PATENT APPLICATION

(11) **EP 4 751 801 A1**
(43) Date of publication of application: **03.06.2026**
(21) Application number: 24884897.0
(22) Date of filing: 31.10.2024
(51) Int. Cl.: B01F 23/45, C10G 19/02, C10G 31/08, C10G 53/02

(54) **MIXER AND ACID-HYDROCARBON SEPARATION SYSTEM AND METHOD**

(30) Priority: 31.10.2023 CN 202311434609
(71) Applicant: China Petroleum & Chemical Corporation, Beijing 100728 (CN); Sinopec Nanjing Research Institute of Chemical Industry Co., Ltd., Nanjing, Jiangsu 210048 (CN); East China University of Science and Technology, Shanghai 200237 (CN)
(72) Inventor: KONG, Weiwei, Nanjing, Jiangsu 210048 (CN); YU, Pinhua, Nanjing, Jiangsu 210048 (CN); REN, Lei, Nanjing, Jiangsu 210048 (CN); HE, Zhiyong, Nanjing, Jiangsu 210048 (CN); JIN, Gang, Nanjing, Jiangsu 210048 (CN); JIANG, Yangyang, Nanjing, Jiangsu 210048 (CN); LI, Haitao, Nanjing, Jiangsu 210048 (CN); LU, Hao, Shanghai 200237 (CN); ZHU, Huatong, Shanghai 200237 (CN); WANG, Yawen, Shanghai 200237 (CN)
(74) Representative: karo IP
(86) International application number: PCT/CN2024/128868
(87) International publication number: WO 2025/092898

(57) **Abstract**

The present invention relates to the technical field of organic matter separation and purification. Disclosed are a mixer and an acid-hydrocarbon separation system and method. The mixer comprises: a housing, wherein a mixing cavity is provided inside the housing, a first liquid inlet through which a raw material liquid enters the mixing cavity is provided on the head end of the housing, and a liquid mixture outlet through which a liquid mixture flows out of the mixing cavity is provided on the tail end of the housing; and a conveying pipe, the conveying pipe running through the mixing cavity via the head end of the housing and extending to the tail end of the housing, wherein a first port of the conveying pipe located at the head end extends out of the housing and forms a second liquid inlet, a second port of the conveying pipe located at the tail end is closed, the part of the conveying pipe located in the mixing cavity forms a spraying section, a plurality of spraying holes distributed at intervals and configured to spray to the mixing cavity a dispersed phase inputted through the second liquid inlet are provided in the pipe wall of the spraying section, and a first helical blade and a second helical blade which are distributed at an interval in the axial direction of the spraying section are provided on the periphery of the spraying section, with the helical direction of the first helical blade being opposite to the helical direction of the second helical blade. During an acid-hydrocarbon separation process, by using the mixer of the present invention, the amount of alkali liquor used in an alkali washing process and the salt content in washing water produced after water washing can be greatly reduced.

## Description

### CROSS-REFERENCE TO RELATED APPLICATIONS

The application claims the benefit of the Chinese patent application No. "202311434609.5", filed on October 31, 2023, the content of which is specifically and entirely incorporated herein by reference.

### TECHNICAL FIELD

The present invention relates to the technical field of organic matter separation and purification, in particular to a mixer and an acid-hydrocarbon separation system and method.

### BACKGROUND ART

In the acid-hydrocarbon separation process, crude separation of an acid-hydrocarbon mixed liquor is first carried out, mainly by means of sedimentation separation or coalescence separation, and the separated oil phase (mainly containing hydrocarbons) is then subjected to washing (e.g., alkali washing and water washing). The washing process is accompanied with a mixing process mainly carried out with a stirred tank or a static mixer. However, the technological process often suffers from several defects. Firstly, the alkali consumption of the alkali washing process is greatly increased due to the high content of residual acids resulting from the incomplete separation of free acid from hydrocarbons during the acid-hydrocarbon separation process; secondly, the alkali consumption of the washing process is largely affected by the mixing effect, but the optimum mixing effect cannot be achieved during the traditional alkali washing process carried out by using a stirred tank or a static mixer, resulting in an increased alkali consumption during the alkali washing process.

### SUMMARY OF THE INVENTION

The present invention aims to overcome the defects in the prior art with respect to the high alkaline consumption during the washing process resulting from the poor mixing effect in the washing process of the acid-hydrocarbon separation, and provides a mixer and an acid-hydrocarbon separation system and method. During an acid-hydrocarbon separation process, by using the mixer of the present invention, the amount of alkali liquor used in an alkali washing process and the salt content in washing water produced after water washing can be greatly reduced.

In order to achieve the above objects, the first aspect of the present invention provides a mixer comprising: a housing, wherein a mixing cavity is provided inside the housing, a first liquid inlet through which a raw material liquid enters the mixing cavity is provided on the head end of the housing, and a liquid mixture outlet through which a liquid mixture flows out of the mixing cavity is provided on the tail end of the housing; and a conveying pipe, the conveying pipe running through the mixing cavity via the head end of the housing and extending to the tail end of the housing, wherein a first port of the conveying pipe located at the head end extends out of the housing and forms a second liquid inlet, a second port of the conveying pipe located at the tail end is closed, the part of the conveying pipe located in the mixing cavity forms a spraying section, a plurality of spraying holes distributed at intervals and configured to spray to the mixing cavity a dispersed phase inputted through the second liquid inlet are provided in the pipe wall of the spraying section, and a first helical blade and a second helical blade which are distributed at an interval in the axial direction of the spraying section are provided on the periphery of the spraying section, with the helical direction of the first helical blade being opposite to the helical direction of the second helical blade.

In some embodiments, the mixing cavity comprises a first variable diameter zone, a buffer zone and a second variable diameter zone distributed in sequence along the direction from the first liquid inlet to the liquid mixture outlet, the first helical blade and the second helical blade are located in a first variable diameter zone and a second variable diameter zone respectively; and/or, the housing is cylindric, an axial direction of the first liquid inlet is perpendicular to an axial direction of the housing, and an axial direction of the liquid mixture outlet is the same as an axial direction of the housing.

In some embodiments, the first variable diameter zone comprises a first mixing section, a first expansion section and a first tapered section sequentially distributed along an axial direction of the spraying section; wherein the first mixing section is in communication with the first liquid inlet, the first expansion section is in communication with the buffer zone; the diameter of the first tapered section gradually decreases along a direction from the first liquid inlet to the liquid mixture outlet, the minimum diameter of the first tapered section is the same as the diameter of the buffer zone, the maximum diameter of the first tapered section is the same as the diameter of the first expansion section, and the diameter of the first expansion section is larger than the diameter of the first mixing section.

In some embodiments, a first radial spacing is provided between a wall surface of the first mixing section and an outer edge of the first helical blade, the minimum radial spacing between the wall surface of the first tapered section and the outer edge of the first helical blade is a second radial spacing, and a third radial spacing is provided between an outer wall surface of the spraying section and the wall surface of the first mixing section, the first radial spacing and the second radial spacing are 1/8 - 1/4 of the third radial spacing.

In some embodiments, a wall surface of the first expansion section is provided with a first spiral linear repoussage encircling the first helical blade, the helical direction of the first spiral linear repoussage is opposite to the helical direction of the first helical blade.

In some embodiments, an axial length of the buffer zone is less than or equal to an axial length of the first mixing section.

In some embodiments, an axial length of the second variable diameter zone is smaller than an axial length of the first variable diameter zone; the second variable diameter zone comprises a second mixing section, a second expansion section and a second tapered section distributed in sequence along an axial direction of the spraying section, the second mixing section is in communication with the buffer zone, the second tapered section is in communication with the liquid mixture outlet; the diameter of the second tapered section gradually decreases along a direction from the second liquid inlet to the liquid mixture outlet, the minimum diameter of the second tapered section is the same as the diameter of the liquid mixture outlet, the maximum diameter of the second tapered section is the same as the diameter of the second expansion section, the diameter of the second expansion section is larger than the diameter of the second mixing section, the diameter of the second mixing section is equal to the diameter of the buffer zone.

In some embodiments, a fourth radial spacing is provided between a wall surface of the second mixing section and an outer edge of the second helical blade, a minimum radial spacing between a wall surface of the second tapered section and an outer edge of the second helical blade is a fifth radial spacing, a sixth radial spacing is provided between an outer wall surface of the spraying section and a wall surface of the second mixing section, the fourth radial spacing and the fifth radial spacing are 1/8 - 1/4 of the sixth radial spacing.

In some embodiments, a wall surface of the second expansion section is provided with a second spiral linear repoussage encircling the second helical blade, the helical direction of the second spiral linear repoussage is opposite to the helical direction of the second helical blade.

In some embodiments, all the spraying holes are distributed in multiple rows and columns on the spraying section, and the number of the spraying holes in the first variable diameter zone is greater than the number of the spraying holes in the second variable diameter zone; in the axial direction of the spraying section, the spacing between two neighboring spraying holes in the buffer zone is smaller than the spacing between two neighboring spraying holes in the second variable diameter zone.

In some embodiments, the pore diameter of the spraying holes gradually decreases from their liquid inlet end to their liquid outlet end, the pore walls of the spraying holes are provided with a third spiral linear repoussage distributed around an axis of the spraying holes, the spraying holes extend obliquely from their liquid inlet end to their liquid outlet end towards the head end of the housing.

The second aspect of the present invention provides acid-hydrocarbon separation system comprising: a sedimentation separation unit, a coalescence separation unit, an alkali washing unit and a water washing unit connected in sequence, the alkali washing unit comprises a first mixer and a first separator connected in sequence, wherein the first mixer is the aforementioned mixer.

The third aspect of the present invention provides an acid-hydrocarbon separation method, the method comprises the following steps:
(1) Subjecting an acid-hydrocarbon mixture to sedimentation separation to obtain a first oil phase;
(2) Subjecting the first oil phase to coalescence separation such that the acid content of the separated second oil phase is 5% or less, preferably 0.1-4.2% of the acid content of the first oil phase;
(3) Performing a first mixing of the second oil phase with an alkali liquor, and subjecting the obtained mixed liquor to a first enhanced separation to obtain a third oil phase;
(4) Subjecting the third oil phase to water washing.

In a mixer according to the present invention, a main phase (e.g., an acid-hydrocarbon mixture) can be introduced into a mixing cavity via a first liquid inlet, a dispersed phase (e.g., an alkali liquor or water) may be introduced into a spraying section of a conveying pipe via a second liquid inlet, and the dispersed phase are sprayed from the spraying holes into the main phase of the mixing cavity, thereby forming a mixed liquor. On the one hand, the mixed liquor will helically flow along a first helical blade and a second helical blade, the helically flowing mixed liquor has a tangential velocity, and shear stress gradients are formed in the mixed liquor such that the dispersed phase is sheared and broken into small droplets, thereby allowing the dispersed phase to be sufficiently blended in the main phase during the rotational flow. On the other hand, when the mixed liquor leaves the region where the first helical blade is located and starts to enter the region where the second helical blade resides, due to the different helical directions of the first helical blade and the second helical blade, the flow state of the mixed liquor will change sharply to form a violent turbulent flow, thereby further enhancing the mixing effect.

Moreover, by using the mixer according to the present invention in an alkali washing process during the acid-hydrocarbon separation, it is possible to sufficiently blend an alkali liquor with the acid-hydrocarbon mixture to obtain the desirable neutralization and deacidification effects, so that the amount of alkali liquor used in an alkali washing process and the salt content in washing water produced after water washing can be greatly reduced.

According to the acid-hydrocarbon separation method of the present invention, the amount of alkali liquor used in the subsequent alkali washing process and the salt content in washing water produced after water washing can be greatly reduced by initially performing a settling separation of the acid-hydrocarbon mixture and then performing a coalescence separation of the oil phase obtained after the settling separation to remove 95% or more of the acid; in addition, by means of the combined operation mode of sufficiently blending and subsequently enhanced separation, the used amount of alkali liquor in the subsequent alkali washing process can be further reduced, the purification effect is further improved, so that the impurity content of the separated purified hydrocarbon product is significantly reduced.

### BRIEF DESCRIPTION OF THE DRAWINGS

FIG. 1 illustrates a schematic diagram of the acid-hydrocarbon separation system of the present invention;
FIG. 2 illustrates a structural schematic diagram of a particle coalescence separation unit in the acid-hydrocarbon separation system of the present invention;
FIG. 3 shows a cross-sectional view of the mixer of the present invention;
FIG. 4 shows a cross-sectional view of the housing in the mixer according to the present invention;
FIG. 5 illustrates an enlarged schematic view of portion A in FIG. 4;
FIG. 6 illustrates an enlarged schematic view of portion B in FIG. 4;
FIG. 7 is a schematic diagram illustrating a distribution of the third spiral linear repoussage in the mixer according to the present invention;
FIG. 8 shows a structural schematic diagram of a separator in the acid-hydrocarbon separation system according to the present invention.

### Description of reference signs

1. Housing;
11. Mixing cavity;
12. First liquid inlet;
13. Liquid mixture outlet;
14. First variable diameter zone;
141. First mixing section;
142. First expansion section;
143. First tapered section;
144. First spiral linear repoussage;
15. Buffer zone;
16. Second variable diameter zone;
161. Second mixing section;
162. Second expansion section;
163. Second tapered section;
164. Second spiral linear repoussage;
2. Conveying pipe;
21. Spraying section;
22. Second liquid inlet;
23. Spraying holes;
24. First helical blade;
25. Second helical blade;
26. Third spiral linear repoussage;
100. Sedimentation separation unit;
200. Particle coalescence separation unit;
2-1. First liquid distributor;
2-2. First bed layer;
2-3. Back flush blocking plate;
2-4. Second bed layer;
2-5. Acid collection structure;
2-6. Support plate;
2-7. First boundary meter;
300. First mixer;
400. First separator;
4-1. Second liquid distributor;
4-2. Rectifier;
4-3. First demulsification separation structure;
4-4. Second demulsification separation structure;
4-5. Third demulsification separation structure;
4-6. Second boundary meter;
500. Second mixer;
600. Second separator.

### DESCRIPTION OF THE PREFERRED EMBODIMENT

The preferred embodiments of the invention will be described in detail below. It should be understood that the specific embodiments described herein merely serve to illustrate and explain the invention, instead of imposing limitations thereto.

In the description of the present application, the terms "first" and "second" are used for descriptive purposes only, and shall not to be construed as indicating relative importance or implying that the number of the indicated technical features. Therefore, unless otherwise specified in the invention, the features defined with the terms "first" and "second" may explicitly or implicitly include one or more of the features; "a plurality of" refers to two or more. The term "comprise" and any variations thereof mean it is not exclusive, there is a possible presence or addition of one or more other features, integers, steps, operations, units, components and/or a combination thereof.

Further, the terms indicating orientations or positional relationships such as "center", "lateral", "upper", "lower", "vertical", "horizontal", "top", "bottom", "inner", "outer" are described in the invention based on the orientation or relative positional relationships shown in the appended figures, the terms are only used for the purpose of facilitating and simplifying the description of the present application, instead of indicating that the mentioned device or element must have a particular orientation, or be constructed and operated in a particular orientation, thus shall not be construed as limitations to the present application.

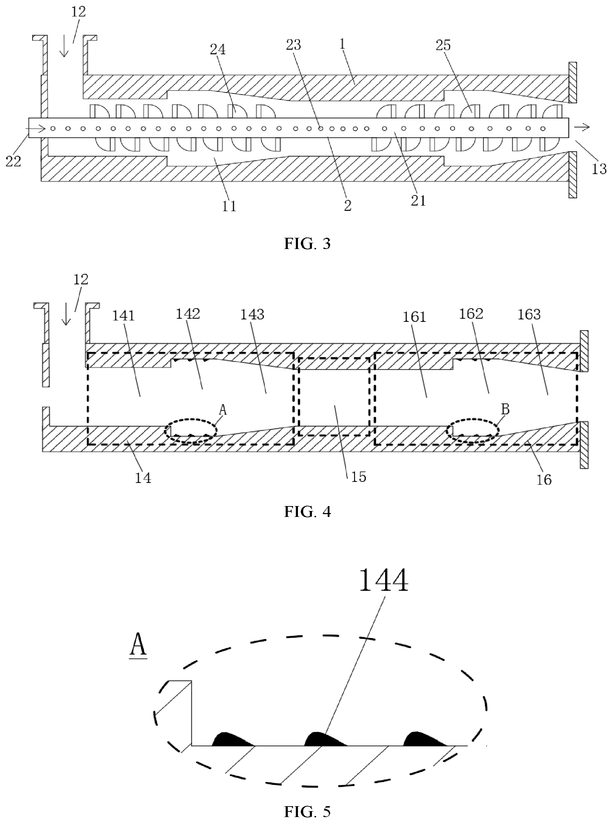

As shown in Figs. 3 and 4, the mixer according to the present invention comprises a housing 1 and a conveying pipe 2, wherein a mixing cavity 11 is provided inside the housing 1, a first liquid inlet 12 through which a raw material liquid enters the mixing cavity 11 is provided on the head end of the housing 1, and a liquid mixture outlet 13 through which a liquid mixture flows out of the mixing cavity 11 is provided on the tail end of the housing 1. A conveying pipe 2 is running through the mixing cavity 11 via the head end of the housing 1 and extending to the tail end of the housing 1, wherein a first port of the conveying pipe 2 located at the head end extends out of the housing 1 and forms a second liquid inlet 22, a second port of the conveying pipe 2 located at the tail end is closed, the part of the conveying pipe 2 located in the mixing cavity 11 forms a spraying section 21, a plurality of spraying holes 23 distributed at intervals and configured to spray to the mixing cavity 11 a dispersed phase inputted through the second liquid inlet 22 are provided in the pipe wall of the spraying section 21, and a first helical blade 24 and a second helical blade 25 which are distributed at an interval in the axial direction of the spraying section 21 are provided on the periphery of the spraying section 21, with the helical direction of the first helical blade 24 being opposite to the helical direction of the second helical blade 25.

In particular, a main phase (e.g., an acid-hydrocarbon mixture) can be introduced into a mixing cavity 11 via a first liquid inlet 12, a dispersed phase (e.g., an alkali liquor or water) may be introduced into a spraying section 21 of a conveying pipe 2 via a second liquid inlet 22, and the dispersed phase are sprayed from the spraying holes 23 into the main phase of the mixing cavity 11, thereby forming a mixed liquor. On the one hand, the mixed liquor will helically flow along a first helical blade 24 and a second helical blade 25, the helically flowing mixed liquor has a tangential velocity, and shear stress gradients are formed in the mixed liquor such that the dispersed phase is sheared and broken into small droplets, thereby allowing the dispersed phase to be sufficiently blended in the main phase during the rotational flow. On the other hand, when the mixed liquor leaves the region where the first helical blade 24 is located and starts to enter the region where the second helical blade 25 resides, due to the different helical directions of the first helical blade 24 and the second helical blade 25, the flow state of the mixed liquor will change sharply to form a violent turbulent flow, thereby further enhancing the mixing effect.

In some embodiments, the helical direction of the first helical blade 24 is toward the first liquid inlet 12, and the helical direction of the first helical blade 24 is toward the liquid mixture outlet 13. In other embodiments, the helical direction of the first helical blade 24 is toward the liquid mixture outlet 13, and the helical direction of the first helical blade 24 is toward the first liquid inlet 12.

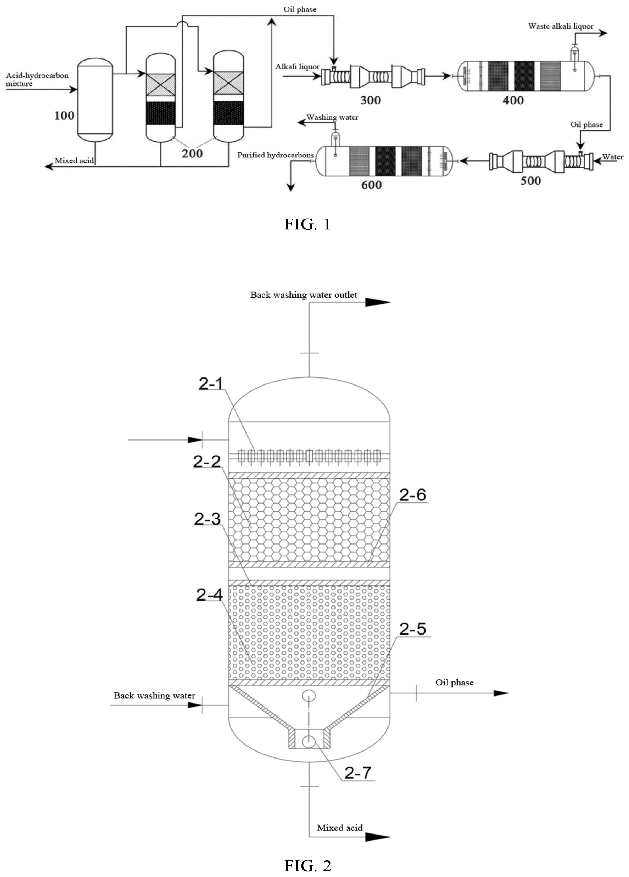

As shown in FIG. 1 and FIG. 2, in some embodiments of the present invention, the mixing cavity 11 comprises a first variable diameter zone 14, a buffer zone 15 and a second variable diameter zone 16 distributed in sequence along the direction from the first liquid inlet 12 to the liquid mixture outlet 13, the first helical blade 24 and the second helical blade 25 are located in a first variable diameter zone 14 and a second variable diameter zone 16 respectively.

Specifically, the first variable diameter zone 14 and the second variable diameter zone 16 may include a plurality of sections with different diameters, the flow state of the mixed liquor in the first variable diameter zone 14 and the second variable diameter zone 16 may be continuously varied to facilitate mixing between the main phase and the dispersed phase.

In some embodiments of the invention, as shown in FIG. 1 and FIG. 2, the housing 1 is cylindric, an axial direction of first liquid inlet 12 is perpendicular to an axial direction of the housing 1, and an axial direction of the liquid mixture outlet 13 is the same as an axial direction of the housing 1.

Specifically, when the main phase enters the mixing cavity 11 via the first liquid inlet 12, the main phase will turn once and form a turbulence, which helps to thoroughly mix the dispersed phase into the main phase. In some embodiments, the ratio of a length of the first helical blade 24 to an axial length of the first variable diameter zone 14 is (0.9-1): 1. Preferably, a length of the first helical blade 24 is equal to an axial length of the first variable diameter zone 14. In some embodiments, the ratio of a length of the second helical blade 25 to an axial length of the second variable diameter zone 16 is (0.9-1): 1. Preferably, a length of the second helical blade 25 is equal to an axial length of the second variable diameter zone 16.

As shown in FIG. 1 and FIG. 2, in some embodiments of the invention, the first variable diameter zone 14 comprises a first mixing section 141, a first expansion section 142 and a first tapered section 143 sequentially distributed along an axial direction of the spraying section 21; wherein the first mixing section 141 is in communication with the first liquid inlet 12, the first expansion section 142 is in communication with the buffer zone 15. The diameter of the first tapered section 143 gradually decreases along a direction from the first liquid inlet 12 to the liquid mixture outlet 13, the minimum diameter of the first tapered section 143 is the same as the diameter of the buffer zone 15, the maximum diameter of the first tapered section 143 is the same as the diameter of the first expansion section 142, and the diameter of the first expansion section 142 is larger than the diameter of the first mixing section 141.

In particular, the first mixing section 141 is cylindric, so that the first mixing section 141 has a consistent diameter, and the environment in which the mixed liquor flows is relatively stable, allowing the mixed liquor in the first mixing section 141 to form a helical flow under the guidance of the first helical blade 24. During the helical flow of the mixed liquor, the mixed liquor has a tangential velocity, and shear stress gradients are formed in the mixed liquor such that the dispersed phase is sheared and broken into small droplets, thereby allowing the dispersed phase to be sufficiently blended in the main phase during the rotational flow. The first expansion section 142 is cylindric, thus it has a consistent diameter, but the diameter of the first expansion section 142 is larger than the diameter of the first mixing section 141. Due to the sudden increase in the diameter of the first expansion section 142, a sudden drop in the flow velocity of the mixed liquor in the first expansion section 142 occurs, and the pressure is raised; in the meanwhile, the flow velocity of the mixed liquor near the wall surface of the first expansion section 142 is low, while the flow velocity of the mixed liquor near the first expansion section 142 is high, i.e., a large flow velocity difference is formed in the first expansion section 142, which allows the mixed liquor in the first expansion section 142 to develop a violent secondary vortex, which breaks and blends the dispersed phase, and causes the dispersed phase to be thoroughly mixed in the main phase. The diameter of the first tapered section 143 gradually decreases, such that the flow velocity of the mixed liquor in the first tapered section will gradually increase, which on the one hand prevents the droplets of the dispersed phase from growing into large size, and on the other hand causes the main phase and the dispersed phase to accelerate and collide in the case of high-velocity flow, and allows the mixed liquor to have a larger flow velocity before entering the second variable diameter zone 16 (facilitates the formation of violent turbulence in the buffer zone 15). Furthermore, the first helical blade 24 can guide the mixed liquor in the first variable diameter zone 14 to always maintain a helical flow, which also facilitates the formation of violent turbulence in the buffer zone 15.

As shown in FIG. 1, in some embodiments of the invention, a first radial spacing is provided between a wall surface of the first mixing section 141 and an outer edge of the first helical blade 24, the minimum radial spacing between the wall surface of the first tapered section 143 and the outer edge of the first helical blade 24 is a second radial spacing, and a third radial spacing is provided between an outer wall surface of the spraying section 21 and the wall surface of the first mixing section 141, the first radial spacing and the second radial spacing are 1/8 - 1/4 of the third radial spacing.

In particular, the spacing between a wall surface of the first mixing section 141 and an outer edge of the first helical blade 24 is small, and the minimum radial spacing between a wall surface of the first tapered section 143 and an outer edge of the first helical blade 24 is small, allowing all the mixed liquor in the first variable diameter zone 14 to flow helically along the first helical blade 24.

As shown in FIG. 3, in some embodiments of the present invention, a wall surface of the first expansion section 142 is provided with a first spiral linear repoussage 144 encircling the first helical blade 24, the helical direction of the first spiral linear repoussage 144 is opposite to the helical direction of the first helical blade 24.

In particular, the first spiral linear repoussage 144 will exert a reverse resistance to the liquid, so that the flow velocity of the mixed liquor at the wall surface of the first expansion section 142 is reduced, and the difference in flow velocity in the first expansion section 142 is enlarged, thereby increasing the severity of the secondary vortex flow, further promoting disruption and mixing of the dispersed phase.

In some embodiments, a radially inner side surface of the first spiral linear repoussage 144 is located between a wall surface of the first expansion section 142 and a wall surface of the first mixing section 141, and a thickness of the first spiral linear repoussage 144 is less than or equal to a wall thickness of the conveying pipe 2.

In some embodiments of the invention, an axial length of the buffer zone 15 is less than or equal to an axial length of the first mixing section 141.

In particular, when the mixed liquor flows from the first variable diameter zone 14 to the second variable diameter zone 16, because the helical direction of the first helical blade 24 is opposite to the helical direction of the second helical blade 25, the liquid needs to change the helical flow direction to flow along the second helical blade 25, and the mixture has a larger flow velocity after being accelerated in the first tapered section 143, thus the flow state of the mixed liquor at the buffer zone 15 will change drastically, which will destroy the original flow state of the dispersed phase and the main phase to form the violent turbulent flow, which promotes thorough mixing of the dispersed phase and the main phase. However, it is desirable that the axial length of the buffer zone 15 is not too long, such that the turbulent flow is concentrated and brings the dispersed phase and the main phase into sufficient contact in a relatively compact area.

In some embodiments of the present invention, an axial length of the second variable diameter zone 16 is smaller than an axial length of the first variable diameter zone 14. The second variable diameter zone 16 comprises a second mixing section 161, a second expansion section 162 and a second tapered section 163 distributed in sequence along an axial direction of the spraying section 21, the second mixing section 161 is in communication with the buffer zone 15, the second tapered section 163 is in communication with the liquid mixture outlet 13. The diameter of the second tapered section 163 gradually decreases along a direction from the second liquid inlet 22 to the liquid mixture outlet 13, the minimum diameter of the second tapered section 163 is the same as the diameter of the liquid mixture outlet 13, the maximum diameter of the second tapered section 163 is the same as the diameter of the second expansion section 162, the diameter of the second expansion section 162 is larger than the diameter of the second mixing section 161, the diameter of the second mixing section 161 is equal to the diameter of the buffer zone 15.

In particular, the second variable diameter zone 16 is located closer to the liquid mixture outlet 13, so that the flow time of the mixed liquor from the second variable diameter zone 16 to the liquid mixture outlet 13 is relatively short, which is disadvantageous for the sufficient mixing of the dispersed phase and the main phase, thus the length of the second variable diameter zone 16 may be relatively short. The second mixing section 161 is cylindric, such that the second mixing section 161 has a consistent diameter, and the environment in which the mixed liquor flows is relatively stable, allowing the mixed liquor in the second mixing section 161 to form a helical flow under the guidance of the second helical blade 25. During the helical flow of the mixed liquor, the mixed liquor has a tangential velocity, and shear stress gradients are formed in the mixed liquor such that the dispersed phase is sheared and broken into small droplets, thereby allowing the dispersed phase to be sufficiently blended in the main phase during the rotational flow. The second expansion section 162 is cylindric, so that the second expansion section 162 has a consistent diameter, but the diameter of the second expansion section 162 is larger than the diameter of the second mixing section 161. Due to the sudden increase of the diameter of the second expansion section 162, a sudden drop in the flow velocity of the mixed liquor in the second expansion section 162 occurs, and the pressure is raised; in the meanwhile, the flow velocity of the mixed liquor near the wall surface of the second expansion section 162 is low, while the flow velocity of the mixed liquor near the spraying section 21 is high, i.e., a large flow velocity difference is formed in the second expansion section 162, which allows the mixed liquor in the second expansion section 162 to develop a secondary vortex that is violent, breaks and blends the dispersed phase, causing the dispersed phase to be thoroughly mixed in the main phase. The diameter of the second tapered section 163 gradually decreases, such that the flow velocity of the mixture in the second expansion section will gradually increase, which on the one hand, prevents the droplets of the dispersed phase from growing into large size, and on the other hand, causes the main phase and the dispersed phase to accelerate and collide in the case of high-velocity flow.

As shown in FIG. 1, in some embodiments of the present invention, a fourth radial spacing is provided between a wall surface of the second mixing section 161 and an outer edge of the second helical blade 25, a minimum radial spacing between a wall surface of the second tapered section 163 and an outer edge of the second helical blade 25 is a fifth radial spacing, a sixth radial spacing is provided between an outer wall surface of the spraying section 21 and a wall surface of the second mixing section 161, the fourth radial spacing and the fifth radial spacing are 1/8 - 1/4 of the sixth radial spacing.

In particular, the spacing between a wall surface of the second mixing section 161 and an outer edge of the second helical blade 25 is small, and the minimum radial spacing between a wall surface of the second tapered section 163 and an outer edge of the second helical blade 25 is small, allowing all the mixed liquor in the second variable diameter zone 16 to helically flow along the second helical blade 25.

As shown in FIG. 4, in some embodiments of the present invention, a wall surface of the second expansion section 162 is provided with a second spiral linear repoussage 164 encircling the second helical blade 25, the helical direction of the second spiral linear repoussage 164 is opposite to the helical direction of the second helical blade 25.

In particular, the second spiral linear playback 164 will exert a reverse resistance to the liquid, so that the flow velocity of the mixed liquor at the wall surface of the second expansion section 162 is reduced, and the difference in flow velocity in the second expansion section 162 is enlarged, thereby increasing the severity of the secondary vortex flow, further promoting the disruption and mixing of the dispersed phase.

In some embodiments, a radially inner side surface of the second spiral linear repoussage 164 is located between a wall surface of the second expansion section 162 and a wall surface of the second mixing section 161, and a thickness of the second spiral linear loudspeaker 164 is less than or equal to a wall thickness of conveying pipe 2.

In some embodiments of the invention, all of the spraying holes 23 are distributed in multiple rows and columns on the spraying section 21 to enable the mixed liquor to be sprayed into various regions of the mixing cavity 11. The second variable diameter zone 16 is closer to the liquid mixture outlet 13, so the flow time of the mixed liquor from the second variable diameter zone 16 to the liquid mixture outlet 13 is relatively short, which is not conducive to the thorough mixing of the main phase with the dispersed phase, thus the amount of spraying holes 23 in the second variable diameter zone 16 may be relatively small, and the amount of spraying holes 23 in the first variable diameter zone 14 may be relatively large. That is, the number of spraying holes 23 in the first variable diameter zone 14 is larger than the spraying holes 23 in the second variable diameter zone 16 to enable more dispersed phase to be thoroughly mixed in the first variable diameter zone 14 and the buffer zone 15 and reduce the dosage of dispersed phase. In the axial direction of the spraying section 21, the spacing between two neighboring spraying holes 23 in the buffer zone 15 is smaller than the spacing between two neighboring spraying holes 23 in the second variable diameter zone 16, the distribution density of the spraying holes 23 in the buffer zone 15 is relatively high to enable the spraying tube to spray more dispersed phase into the buffer zone 15. Because of the violent turbulent flow is formed in the buffer zone 15, the main phase is more easily contacted with the dispersed phase, thus the mixing is more thorough.

As shown in FIG. 5, in some embodiments of the present invention, the pore diameter of the spraying holes 23 gradually decreases from their liquid inlet end to their liquid outlet end, the pore walls of the spraying holes 23 are provided with a third spiral linear repoussage 26 distributed around an axis of the spraying holes 23, the spraying holes 23 extend obliquely from their liquid inlet end to their liquid outlet end towards the head end of the housing 1.

In particular, the spraying holes 23 are tapered holes, the dispersed phase will have a certain acceleration in the spraying holes 23, increasing the impact of the dispersed phase on the mixed liquor in the mixing cavity 11, enhancing the disturbance to the mixed liquor; moreover, the third spiral linear repoussage 26 can direct the dispersed phase to helically flow, further increasing the disturbance to the mixed liquor, thereby achieving an adequate mixing of the main phase with the dispersed phase.

As shown in FIGS. 1-3 and 8, an acid-hydrocarbon separation system according to the present invention comprises: a sedimentation separation unit 100, a coalescence separation unit, an alkali washing unit and a water washing unit connected in sequence, the alkali washing unit comprises a first mixer 300 and a first separator 400 connected in sequence, wherein the first mixer 300 is the aforementioned mixer.

In the acid-hydrocarbon separation system according to the present invention, the initial separation of the mixed acids in the acid-hydrocarbon mixture from the hydrocarbons can be carried out by the sedimentation separation unit 100, a part of the mixed acids are separated and then discharged, and the separated first oil phase enters the subsequent coalescence separation unit for further processing.

In the acid-hydrocarbon separation system according to the invention, the sedimentation separation unit 100 may be a gravity settling tank. In a more preferred embodiment, an interior of the sedimentation separation unit 100 is provided with a corrugated plate filler, the feed port of the acid-hydrocarbon mixture is arranged above the corrugated plate filler, the separated mixed acids after the sedimentation separation are discharged from the bottom of the sedimentation separation unit 100, and the separated first oil phase is discharged from the upper outlet and enters a subsequent coalescence separation unit for further treatment. Further preferably, the corrugation structure on the corrugated plate filling has an intersection angle of 50°-70° relative to the horizontal direction. In the text, an intersection angle of the corrugated structure relative to the horizontal direction refers to an angle of the inclined plane of the corrugated plate relative to the horizontal direction.

In the acid-hydrocarbon separation system according to the invention, in a case of preferably, the coalescence separation unit comprises one or more particle coalescence separation units 200, an inner cavity of the particle coalescence separation units 200 is provided with a particle bed layer formed by media particles, which are hydrophilic particles, or a combination of hydrophilic particles and hydrophobic particles. In the present invention, the particle coalescence separation unit 200 is used for treating the first oil phase separated from the sedimentation separation unit 100, it can sufficiently remove acid droplets with a diameter of 15µm or more (removal rate of 95% or more), and remove most of the acid droplets with a diameter less than 15µm (removal rate of 50% or more).

In some embodiments, the particle bed layer comprises two or more layers, in particular, for example, it may be two, three, or four layers. Each particle bed layer may be formed by packing of media particles having the same or different morphology and different hydrophilicity and hydrophobicity, and/or each particle bed layer may be formed by filling the media particles having a single size or media particles having different sizes respectively.

In the present invention, the particle diameter of the media particles may be within the range of 0.2-5mm, preferably within the range of 0.5-3mm. The shape of the media particles may be various regular three-dimensional structures (e.g., spherical shape), or various irregular structures.

In the present invention, the hydrophilic particles include but are not limited to at least one of ore, ceramic and glass.

In the present invention, the hydrophobic particles include but are not limited to at least one of polypropylene, polystyrene and polyurethane.

In a preferred embodiment, the acid-hydrocarbon separation system includes two or more particle coalescence separation units 200 connected in parallel with each other. According to the preferred embodiment, two or more of the particle coalescence separation units 200 may be operated simultaneously, or in a state that one unit is operated and another unit is standby, during an operation process of the device, when the pressure drop of one of the particle coalescence separation units is high, a large amount of accumulated particle suspension is intercepted in the surface of bed layer, under the circumstance, the corresponding particle coalescence separation unit may be closed and back flushed to release the particle suspension accumulated in the bed layer, and the other particle coalescence separation units can be activated such that the whole treatment process can be continuously performed. In particular, each of the particle coalescence separation units has a working pressure of 0.01-0.1MPa and a cross-sectional flow velocity of 0.001-0.02m/s, the corresponding particle coalescence separation unit is back flushed when the pressure drop of the particle coalescence separation unit is higher than 0.08MPa.

In a more preferred embodiment, the particle coalescence separation unit 200 is a vertical separator as shown in FIG. 2, an inner cavity of the particle coalescence separation unit 200 is provided with a first liquid distributor 2-1, two or more particle bed layers, and an acid collection structure 2-5 arranged from top to bottom, the first oil phase is injected via an inlet disposed above the first liquid distributor 2-1, the two or more particle bed layers are used for capture and coalescence of emulsified acid droplets in the first oil phase, and retention of solid suspension on a surface of the particle bed layer, the oil phase processed by the particle bed layer is discharged through an outlet disposed at a lower part of the particle bed layer and enters the subsequent separation process for further treatment, and the separated mixed acids are discharged via an outlet at the bottom of the acid collection structure 2-5. In a specific embodiment, two bed layers are arranged in the particle coalescence separation unit 200, namely a first bed layer 2-2 and a second bed layer 2-4, both of the first bed layer 2-2 and the second bed layer 2-4 are provided with a support plate 2-6 at the bottom, both of the first bed layer 2-2 and the second bed layer 2-4 are provided with a back flush blocking plate 2-3 at the top, and a certain spacing is arranged between the first bed layer 2-2 and the second bed layer 2-4, a backwash water inlet is disposed at the lower part of the second bed layer 2-4, and backwash water outlet is disposed at the upper part of the first bed layer 2-2. The backwash water during the back flush process can be provided by the washing water separated from a subsequent water washing unit.

In the particle coalescence separation unit 200, the first liquid distributor 2-1 may have a distribution disc structure.

In the particle coalescence separation unit 200, the acid collection structure 2-5 may be a Y-type collector for collecting the acid phases (i.e. the mixed acids) aggregated and separated by the bed layers.

In the particle coalescence separation unit 200, in a case of preferably, the bottom outlet of the acid collection structure 2-5 is provided with a first boundary meter 2-7 for monitoring the phase interface of the oil phase and the acid phase, as shown in FIG. 2.

In the acid-hydrocarbon separation system of the present invention, in a case of preferably, the coalescence separation unit further comprises one or more fiber coalescence separation units, the fiber coalescence separation unit and the particle coalescence separation unit (200) are connected in series with each other, an inner cavity of the fiber coalescence separation unit is provided with a fiber bed layer formed by fiber media comprising hydrophilic fibers and hydrophobic fibers. Further preferably, the fiber coalescence separation units are located downstream the particle coalescence separation unit 200 for performing the further deacidification on the oil phase obtained after treatment with the particle coalescence separation unit 200.

In a more preferred embodiment, the fiber bed layer is formed by stacking a plurality of fiber layers, each of the fiber layers is woven from hydrophilic fibers and hydrophobic fibers. The hydrophilic fibers may be metal fibers (e.g., monel alloy). For example, the material of hydrophobic fibers may be at least one selected from the group consisting of Teflon, polyvinyl chloride and polyphenylene sulphide.

In the acid-hydrocarbon separation system of the present invention, the alkali washing unit comprises a first mixer 300 and a first separator 400 connected in series. In the alkali washing unit, the first mixer 300 is used for thoroughly mixing the oil phase separated from the particle coalescence separation unit 200 or the oil phase separated from the fiber coalescing unit with the alkali liquor, hydrocarbon droplets are encapsulated with water-soluble aqueous phase droplets or alternately encapsulated with multi-layer liquid phase, in order to disassemble the multi-layer liquid phase package of the non-soluble droplets and aggregate the same phase droplets, the mixed liquor formed after mixing the oil phase with the alkali liquor is then demulsified, aggregated and formed into a liquid phase delamination by the first separator 400, waste alkali liquor and neutralized products are introduced into the water-soluble phase disposed at the upper layer, and the hydrocarbons are introduced into the oil phase located at the lower layer.

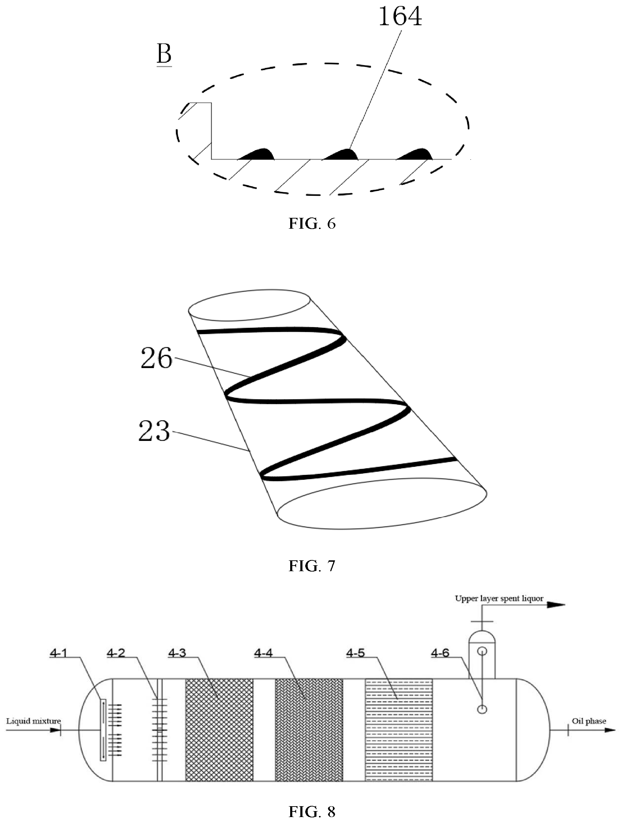

In a more preferred embodiment, as shown in FIG. 8, the first separator 400 comprises: a second housing; and a second liquid distributor 4-1, a rectifier 4-2, a first demulsification separation structure 4-3, a second demulsification separation structure 4-4 and a third demulsification separation structure 4-5 sequentially arranged in an inner cavity of the second housing along a material flow direction, wherein the first demulsification separation structure 4-3 is a layer of hydrophilic fibers, the second demulsification separation structure 4-4 is an interlaced layer of lipophilic fibers and hydrophilic fibers, and the third demulsification separation structure 4-5 is a corrugated plate. With such a structure configuration, the second liquid distributor 4-1 and the rectifier 4-2 can further disperse the mixed liquor obtained after the mixing process; the first demulsification separation structure 4-3 can demulsify and separate the hydrocarbon liquid droplets from the water-soluble phase droplets in the mixed liquor, for example, two-layer droplets of hydrocarbon-in-water or water-in-hydrocarbon are separated, or three-layer droplets are subjected to separation of one layer to form a two-layer droplets; the second demulsification separation structure 4-4 can further separate the water-soluble phase droplets from the hydrocarbon liquid droplets which are not completely separated; the third demulsification separation structure 4-5 allows the water-soluble phase droplets and the hydrocarbon liquid droplets to separate sufficiently and form a liquid phase sub-layer; therefore, according to the above preferred embodiment, the separation efficiency of the hydrocarbons from other components (e.g., acid, alkali, salt, water, etc.) can be further enhanced, so that the water content of the separated oil phase is 400mg/L or less.

In the first separator 400, the second liquid distributor 4-1 may be a pipe-type liquid distributor or a disc-type liquid distributor.

In the first separator 400, the rectifier 4-2 preferably has a flat plate perforation structure, wherein the pore size may be within the range of 1-3mm, the pore center-to-center spacing may be within the range of 5-8mm, and the perforation rate may be within the range of 20%-40%.

In the first separator 400, the angle between the corrugation structure on the corrugated plate and the horizontal direction may be 30°-60°.

In the first separator 400, in order to further improve the delamination effect of the water-soluble phase droplets and the hydrocarbon droplets, it is preferable that the corrugated plate is formed by alternating stacking of oleophilic materials and hydrophilic materials. More preferably, both the peaks and valleys of the corrugated plate are provided with holes. Further preferably, the number of holes at peaks and the number of holes at valleys are equal, and the specific number of holes can be adjusted according to the actual conditions of the material to be separated. With such a structure configuration, small droplets after demulsification can quickly aggregate and float upward, floating upward of the water-soluble phase droplets and separation of the hydrocarbon droplets are accelerated, and the flow field of the two-phase fluid is more stable.

In the first separator 400, after the formation of the liquid phase stratification, a water-soluble phase (e.g., a waste alkali liquor phase or a water-soluble phase containing salts) is positioned in an upper layer and discharged via a waste liquor outlet, and an oil phase is positioned in a lower layer and discharged through an oil phase outlet. In a case of preferably, a second boundary meter 4-6 is disposed at the waste liquor outlet of the first separator 400 for monitoring the phase interfaces of the water-soluble phase and the oil phase.

In the acid-hydrocarbon separation system according to the present invention, the water washing unit may be a conventional water washing apparatus in the field. In a case of preferably, the water washing unit comprises a second mixer 500 and a second separator 600 connected with each other. In the water washing unit, the oil phase obtained after an alkali washing process may further contain some water-soluble mixtures (e.g., salts, etc.). The hydrocarbons containing salts may bring forth an explosion risk in the subsequent production process, thus it is necessary to perform water washing to remove salts.

In the acid-hydrocarbon separation system according to the invention, the second mixer 500 and the first mixer 300 may have the same or different structures, preferably the same structure.

In the acid-carbohydrate separation system according to the present invention, the second separator 600 and the first separator 400 may have the same or different structures, preferably the same structure.

The acid-carbohydrate separation method according to the present invention comprises the following steps:
(1) Subjecting an acid-hydrocarbon mixture to sedimentation separation to obtain a first oil phase;
(2) Subjecting the first oil phase to coalescence separation to obtain a second oil phase;
(3) Performing a first mixing of the second oil phase with an alkali liquor, and subjecting the obtained mixed liquor to a first enhanced separation to obtain a third oil phase;
(4) Subjecting the third oil phase to water washing.

In the method of the present invention, the sedimentation separation process in step (1) may be performed to primarily separate the acids from the hydrocarbons in the acid-hydrocarbon mixture, a part of the acids is separated and then discharged, and the separated first oil phase is subjected to further treatment in a subsequent separation process.

In the method of the present invention, the sedimentation separation process of step (1) may be performed in a segment separation unit. In some embodiments, an interior of the sedimentation separation unit is provided with a corrugated plate filler, the feed port of the acid-hydrocarbon mixture is arranged above the corrugated plate filler, the separated acids after the sedimentation separation are discharged from the bottom of the sedimentation separation unit, and the separated first oil phase is discharged from the upper outlet and enters a subsequent separation process for further treatment.

In a case of preferably, the corrugation structure on the corrugated plate filler has an intersection angle of 50°-70° relative to the horizontal direction.

In the present invention, the sedimentation separation unit may be a gravity settling tank.

In the method of the present invention, the coalescence separation process in step (2) causes that the acid content of the separated second oil phase is 5% or less, preferably within the range of 0.1-4.2%, of the acid content of the first oil phase. Specifically, for example, the proportion may be 0.1%, 0.5%, 1%, 1.2%, 1.5%, 1.8%, 2%, 2.2%, 2.5%, 2.8%, 3%, 3.2%, 3.5%, 3.6%, 3.7%, 3.8%, 3.9%, 3.95%, 4%, 4.05%, 4.1%, 4.15%, or 4.2%.

In the method of the present invention, the coalescence separation process in step (2) preferably comprises:

contacting the first oil phase with media particles for carrying out a first coalescence separation, wherein the media particles are hydrophilic particles, or a combination of hydrophilic particles and hydrophobic particles;

optionally, the oil phase obtained from the first coalescence separation contacts with a fiber media for performing a second coalescence separation, wherein the fiber media comprises hydrophilic fibers and hydrophobic fibers.

The first coalescence separation process can sufficiently remove acid droplets with a diameter of 15µm or more (removal rate of 95% or more), and remove most of the acid droplets with a diameter less than 15µm (removal rate of 50% or more).

In a case of preferably, the first coalescence separation process comprises: passing the first oil phase from top to bottom through a particle bed layer formed from the media particles.

In the invention, the media particles may have a particle diameter within the range of 0.2-5mm, preferably within the range of 0.5-3mm. The shape of the media particles may be various regular three-dimensional structures (e.g., spherical shape), or various irregular structures.

In the invention, the hydrophilic particles are at least one selected from the group consisting of ore particles, ceramic particles and glass particles.

In the present invention, the hydrophobic particles include but are not limited to at least one selected from the group consisting of polypropylene particles, polystyrene particles, and polyurethane particles.

In the method of the present invention, in a case of preferably, the first coalescence separation process in step (2) is performed in two or more particle coalescence separation units connected in parallel, each of the particle coalescence separation units is packed with a particle bed layer formed of the medium particles, each of the particle coalescence separation units has a working pressure of 0.01-0.1MPa and a cross-sectional flow velocity of 0.001-0.02m/s, the corresponding particle coalescence separation unit is back flushed when the pressure drop of the particle coalescence separation unit is higher than 0.08MPa. According to the above preferred embodiment, two or more of the particle coalescence separation units may be operated simultaneously, or in a state that one unit is operated and another unit is standby, during an operation process of the device, when the pressure drop of one of the particle coalescence separation units is high, a large amount of accumulated particle suspension is intercepted in the surface of bed layer, under the circumstance, the corresponding particle coalescence separation unit may be closed and back flushed to release the particle suspension accumulated in the bed layer, and the other particle coalescence separation units can be activated such that the whole treatment process can be continuously performed.

In a more preferred embodiment, the particle coalescence separation unit 200 is a vertical separator as shown in FIG. 2, an inner cavity of the particle coalescence separation unit 200 is provided with a first liquid distributor 2-1, two or more particle bed layers, and an acid collection structure 2-5 arranged from top to bottom, the first oil phase is injected via an inlet disposed above the first liquid distributor 2-1, the two or more particle bed layers are used for capture and coalescence of emulsified acid droplets in the first oil phase, and retention of solid suspension on a surface of the particle bed layer, the oil phase processed by the particle bed layer is discharged through an outlet disposed at a lower part of the particle bed layer and enters the subsequent separation process for further treatment, and the separated mixed acids are discharged via an outlet at the bottom of the acid collection structure 2-5. In a specific embodiment, two bed layers are arranged in the particle coalescence separation unit 200, namely a first bed layer 2-2 and a second bed layer 2-4, both of the first bed layer 2-2 and the second bed layer 2-4 are provided with a support plate 2-6 at the bottom, both of the first bed layer 2-2 and the second bed layer 2-4 are provided with a back flush blocking plate 2-3 at the top, and a certain spacing is arranged between the first bed layer 2-2 and the second bed layer 2-4, and a backwash water inlet is disposed at the lower part of the second bed layer 2-4, and backwash water outlet is disposed at the upper part of the first bed layer 2-2. The backwash water during the back flush process can be provided by the washing water separated from a subsequent water washing unit.

In the particle coalescence separation unit 200, the first liquid distributor 2-1 may have a distribution disc structure.

In the particle coalescence separation unit 200, the acid collection structure 2-5 may be a Y-type collector for collecting the acid phases (i.e. the mixed acids) aggregated and separated by the bed layers.

In the particle coalescence separation unit 200, in a case of preferably, the bottom outlet of the acid collection structure 2-5 is provided with a first boundary meter 2-7 for monitoring the phase interface of the oil phase and the acid phase, as shown in FIG. 2.

In the method of the invention, the acids may be further removed by the second coalescence separation. In a case of preferably, the second coalescence separation process comprises: passing the oil phase obtained from the first coalescence separation from top to bottom through a fiber bed layer formed of the fiber media. The fiber bed layer is formed by stacking a plurality of fiber layers, each of the fiber layers is woven from hydrophilic fibers and hydrophobic fibers. The hydrophilic fibers may be metal fibers (e.g., monel alloy). For example, the material of hydrophobic fibers may be at least one selected from the group consisting of Teflon, polyvinyl chloride and polyphenylene sulphide.

In the method of the invention, in step (3), the second oil phase separated from the coalescence separation process is mixed with an alkali liquor, the mixed liquor obtained following the mixing process is then subjected to an enhanced separation. The mixing process can be performed such that the second oil phase is sufficiently mixed with the alkali liquor for carrying out the neutralization reaction. The enhanced separation process may cause that the mixed liquor is subjected to demulsification and liquid phase delamination, the product obtained following the acid mixing and alkali washing process is separated from hydrocarbons, thereby achieving the sufficient deacidification. In step (3), by sufficiently mixing the second oil phase with the alkali liquor, hydrocarbon droplets are encapsulated with water-soluble aqueous phase droplets or alternately encapsulated with multi-layer liquid phase, in order to disassemble the multi-layer liquid phase package of the non-soluble droplets and aggregate the same phase droplets, the mixed liquor formed after mixing the oil phase with the alkali liquor is then demulsified, aggregated and formed into a liquid phase delamination by the first separator 400, waste alkali liquor and neutralized products are introduced into the water-soluble phase disposed at the upper layer, and the hydrocarbons are introduced into the oil phase located at the lower layer.

In a case of preferably, the first mixing process in step (3) makes the median particle diameter of the hydrocarbon dispersed phase droplets in the mixed liquor obtained after mixing is 100µm or less, more preferably 40µm or less (e.g., 10-40µm). A smaller median particle diameter of the hydrocarbon dispersed phase droplets in the mixed liquor indicates a more thorough mixing of the oil phase and the alkali liquor. In a more preferred embodiment, the first mixing process is carried out in the aforementioned mixer, according to the embodiment, the thorough mixing of the alkali liquor and the acid-hydrocarbon mixture is obtained with the desirable neutralization and deacidification effects, thus the amount of alkali liquor used in an alkali washing process and the salt content in washing water produced after water washing can be greatly reduced.

In a case of preferably, the first enhanced separation process in step (3) causes that the separated third oil phase has a water content being 600mg/L or less, more preferably 400mg/L or less. (e.g., 200-400mg/L). The lower the water content of the oil phase indicates the better desalination and dealkalization effects. In some embodiments, the first enhanced separation process is carried out in a horizontal separator as shown in FIG. 8, the horizontal separator comprises a second housing; and a second liquid distributor 4-1, a rectifier 4-2, a first demulsification separation structure 4-3, a second demulsification separation structure 4-4, a woven layer of lipophilic and hydrophilic fibers and a third demulsification separation structure 4-5 sequentially arranged in the inner cavity of the second housing along a material flow direction, wherein the first demulsification separation structure 4-3 is a layer of hydrophilic fibers, the second demulsification separation structure 4-4 is an interlaced layer of lipophilic fibers and hydrophilic fibers, and the third demulsification separation structure 4-5 is a corrugated plate. With such a structure configuration, the second liquid distributor 4-1 and the rectifier 4-2 can further disperse the mixed liquor obtained after the mixing process; the first demulsification separation structure 4-3 can demulsify the hydrocarbon liquid droplets in the mixed liquor and separate from the water-soluble phase droplets, for example, two-layer droplets of hydrocarbon-in-water or water-in-hydrocarbon are separated, or three-layer droplets are subjected to separation of one layer to form a two-layer droplets; the second demulsification separation structure 4-4 can further separate the water-soluble phase droplets from the hydrocarbon liquid droplets which are not completely separated; the third demulsification separation structure 4-5 allows the water-soluble phase droplets and the hydrocarbon liquid droplets to separate sufficiently and form a liquid phase sub-layer; therefore, according to the above preferred embodiment, the separation efficiency of the hydrocarbons from other components (e.g., acid, alkali, salt, water, etc.) can be further enhanced, so that the water content of the separated oil phase is 400mg/L or less.

In the horizontal separator, the second liquid distributor 4-1 may be a pipe-type liquid distributor or a disc-type liquid distributor.

In the horizontal separator, the rectifier 4-2 preferably has a flat plate perforation structure, wherein the pore size may be within the range of 1-3mm, the pore center-to-center spacing may be within the range of 5-8mm, and the perforation rate may be within the range of 20%-40%.

In the horizontal separator, the angle between the corrugation structure on the corrugated plate and the horizontal direction may be 30°-60°.

In the horizontal separator, in order to further improve the delamination effect of the water-soluble phase droplets and the hydrocarbon droplets, it is preferable that the corrugated plate is formed by alternating stacking of oleophilic materials and hydrophilic materials. More preferably, both the peaks and valleys of the corrugated plate are provided with holes. Further preferably, the number of holes at peaks and the number of holes at valleys are equal, and the specific number of holes can be adjusted according to the actual conditions of the material to be separated. With such a structure configuration, small droplets after demulsification can quickly aggregate and float upward, floating upward of the water-soluble phase droplets and separation of the hydrocarbon droplets are accelerated, and the flow field of the two-phase fluid is more stable.

In the horizontal separator, after the formation of the liquid phase stratification, a waste alkali liquor phase is positioned in an upper layer and discharged via a waste liquor outlet, and an oil phase is disposed in a lower layer and discharged through an oil phase outlet. In a case of preferably, a second boundary meter 4-6 is disposed at the waste liquor outlet of the horizontal separator for monitoring the phase interfaces of the water-soluble phase and the oil phase.

In the method of the present invention, the volumetric flow ratio of the second oil phase to the alkali liquor in step (3) may be 1: (0.05-0.2), preferably 1: (0.05-0.18), more preferably 1: (0.08-0.15), further preferably 1: (0.08-0.12).

In the method of the invention, the alkali liquor may be at least one of a sodium hydroxide solution, a potassium hydroxide solution and aqueous ammonia. The alkali liquor may have a concentration within the range of 10-40wt%, preferably within the range of 15-35wt%.

In the method of the present invention, the oil phase obtained after an alkali washing process may further contain some water-soluble mixtures (e.g., salts, etc.). The hydrocarbons containing salts may bring forth an explosion risk in the subsequent production process, thus it is necessary to perform water washing to remove salts.

In the method of the present invention, the water washing process in step (4) preferably comprises: performing a second mixing on the third oil phase with water, and subjecting the obtained mixed liquor to a second enhanced separation. By mixing the third oil phase with water, hydrocarbon droplets are encapsulated with water-soluble aqueous phase droplets or alternately encapsulated with multi-layer liquid phase, in order to disassemble the multi-layer liquid phase package of the non-soluble droplets and aggregate the same phase droplets, it is necessary to perform an enhanced separation on the mixed liquor, such that the mixed liquor is demulsified, aggregated and formed into a liquid phase delamination, the salts and the like are introduced into the water-soluble phase disposed at the upper layer, and the purified hydrocarbons are disposed at the lower layer.

In a case of preferably, the second mixing process makes the median particle diameter of the hydrocarbon dispersed phase droplets in the mixed liquor obtained after mixing is 100µm or less, more preferably 40µm or less (e.g., 10-40µm). A smaller median particle diameter of the hydrocarbon dispersed phase droplets in the mixed liquor indicates that the crude nitrobenzene is more sufficiently mixed with water.

In the present invention, the mixing unit used in the second mixing process and the mixing unit used in the first mixing process may be the same or different, preferably the same. In a case of preferably, the mixing unit used in the second mixing process is the horizontal mixer shown in FIG. 3.

In a case of preferably, the second enhanced separation process causes that the separated purified hydrocarbon has a water content being 600mg/L or less, more preferably 400mg/L or less (e.g. 200-400mg/L). The lower water content of the purified hydrocarbons reduces the energy consumption in the subsequent rectification system.

In the present invention, the separator used during the second enhanced separation and the separator used during the first enhanced separation may be the same or different, preferably the same. In a case of preferably, the separator used during the second enhanced separation is the horizontal separator shown in FIG. 4.

In the method of the present invention, during the water washing process, the volumetric flow ratio of the third oil phase to water is 1: (0.1-0.6), preferably 1: (0.1-0.5), more preferably 1: (0.15-0.4), further preferably 1: (0.2-0.3).

In the method of the present invention, the acid in the acid-hydrocarbon mixture may be at least one selected from the group consisting of hydrochloric acid, sulfuric acid, nitric acid, phosphoric acid, hydrofluoric acid and hydrobromic acid.

In the method of the invention, the hydrocarbon in the acid-hydrocarbon mixture may be aromatic hydrocarbon, preferably at least one selected from the group consisting of nitroaromatic hydrocarbon (e.g., nitrobenzene, nitrotoluene), halogenated aromatic hydrocarbon, and nitro-halogenated aromatic hydrocarbon (e.g., nitrochlorobenzene).

In some embodiments, the acid-hydrocarbon mixture is a nitration reaction product (i.e., a nitration liquid), wherein the hydrocarbon to be separated and purified is nitrobenzene. The separation and purification of the nitration reaction product are performed according to the acid-hydrocarbon separation method of the invention, the amount of alkali liquor used in an alkali washing process and the salt content in washing water produced after water washing can be greatly reduced, and the content of nitrophenol in the finally separated purified nitrobenzene is significantly reduced.

In other embodiments, the acid-hydrocarbon mixture is a nitration reaction product comprising nitrotoluene, wherein the hydrocarbon to be separated and purified is nitrotoluene. The nitration reaction product comprising nitrotoluene may be separated and purified according to the method in the invention, the amount of alkali liquor used in an alkali washing process and the salt content in washing water produced after water washing can be greatly reduced.

In other embodiments, the acid-hydrocarbon mixture is a nitration reaction product containing nitrochlorobenzene, wherein the hydrocarbon to be separated and purified is nitrochlorobenzene. The nitration reaction product containing nitrochlorobenzene is separated and purified according to the acid-hydrocarbon separation method in the invention, the amount of alkali liquor used in an alkali washing process and the salt content in washing water produced after water washing can be greatly reduced.

The mixer and acid-hydrocarbon separation system and method according to the present invention will be further described with reference to examples. The examples are implemented under the premise of the technical scheme of the present invention, and provide the detailed embodiments and specific operation processes, but the protection scope of the present invention is not limited to the examples described below.

Unless otherwise specified in the invention, the experimental methods in the examples below were conventional methods in the art. Unless otherwise specified in the invention, the experimental materials used in the examples below were commercially available.

In the following examples and comparative examples, the amounts of acids in the first oil phase obtained after the sedimentation separation and the second oil phase obtained after the coalescence separation were determined according to the acid-base titration method;
The median particle diameter of the hydrocarbon dispersed phase droplets in the mixed liquor obtained from the first mixer and the median particle diameter of the hydrocarbon dispersed phase droplets in the mixed liquor obtained from the second mixer were measured by using an electron microscope and a particle size counter;
The water content of the third oil phase separated from the first separator and the water content of the finally separated purified hydrocarbons were determined using a Karl Fischer moisture analyzer;
The nitrophenol content of the final separated purified hydrocarbons was determined by using the spectrophotometry;
The salt content of washing water generated during the water washing process was measured by using an ion chromatography.

### Example 1

### (1) Acid-hydrocarbon separation system

The structure of the acid-hydrocarbon separation system used in the example was as shown in FIG. 1-8, specifically, as shown in FIG. 1, the acid-hydrocarbon separation system included a sedimentation separation unit 100, two particle coalescence separation units 200 connected in parallel, a first mixer 300, a first separator 400, a second mixer 500, and a second separator 600; the sedimentation separation unit 100 was a gravity settling tank, and a corrugated plate filler was arranged in the gravity settling tank; the particle coalescence separation unit 200 was a vertical separator shown in FIG. 2, the inner cavity of the vertical separator was sequentially provided with a first liquid distributor 2-1, two bed layers (namely a first bed layer 2-2 and a second bed layer 2-4) formed by medium particles, and an acid collection structure 2-5 from top to bottom, the top of each bed layer was provided with a back flush blocking plate 2-3, the bottom of each bed layer was provided with a support plate 2-6, an oil phase inlet was arranged above the first liquid distributor 2-1, the oil phase inlet was in communication with an oil phase outlet of the sedimentation separation unit 100, the bottom of the acid collection structure 2-5 was provided with an outlet for discharging mixed acids, an oil phase outlet was arranged at the lower part of the second bed layer 2-4 (positioned at the side of the device), a backwash water inlet was disposed at the lower part of the second bed layer 2-4, a backwash water outlet was disposed at the upper part of the first bed layer 2-2; the first mixer 300 and the second mixer 500 were the same, both were horizontal mixers shown in FIG. 3, which included a housing 1 and a conveying pipe 2, wherein the mixing cavity 11 was provided inside the housing 1, a first liquid inlet 12 through which a raw material liquid entered the mixing cavity 11 was provided on the head end of the housing 1, and a liquid mixture outlet 13 through which a liquid mixture flowed out of the mixing cavity 11 was provided on the tail end of the housing 1. A conveying pipe 2 run through the mixing cavity 11 via the head end of the housing 1 and extended to the tail end of the housing 1, wherein a first port of the conveying pipe 2 located at the head end extended out of the housing 1 and formed a second liquid inlet 22, a second port of the conveying pipe 2 located at the tail end was closed, the part of the conveying pipe 2 located in the mixing cavity 11 formed a spraying section 21, a plurality of spraying holes 23 distributed at intervals and configured to spray to the mixing cavity 11 a dispersed phase inputted through the second liquid inlet 22 were provided in the pipe wall of the spraying section 21, and a first helical blade 24 and a second helical blade 25 which were distributed at an interval in the axial direction of the spraying section 21 were provided on the periphery of the spraying section 21, with the helical direction of the first helical blade 24 that was opposite to the helical direction of the second helical blade 25; the first liquid inlet 12 of the first mixer 300 was in communication with an oil phase outlet of the particle coalescence separation unit 200, the second liquid inlet 22 of the first mixer 300 was used for injecting an alkali liquor; the second liquid inlet 22 of the second mixer 500 was used for injecting water; the first separator 400 and the second separator 600 were the same, both were the horizontal separator shown in FIG. 8, and comprised a housing, and a second liquid distributor 4-1, a rectifier 4-2, a first demulsification separation structure 4-3, a second demulsification separation structure 4-4 and a third demulsification separation structure 4-5 sequentially arranged in the inner cavity of the housing along a material flow direction, wherein the first demulsification separation structure 4-3 was a layer of hydrophilic fibers, the second demulsification separation structure 4-4 was an interlaced layer of lipophilic fibers and hydrophilic fibers, and the third demulsification separation structure 4-5 was a corrugated plate; the first separator 400 was provided with a liquid inlet, an oil phase outlet and a waste alkali liquor outlet for discharging the waste alkali liquor, the second separator 600 was provided with an oil phase outlet and a washing water outlet for discharging the washing water, wherein the liquid mixture outlet 13 of the first separator 300 was in communication with the liquid inlet of the first separator 400, the oil phase outlet of the first separator 400 was in communication with the first liquid insert 12 of the second separator 500, and the liquid mixture outlet 13 of the second separator 500 was in communication with the liquid inlet of the second separator 600.

### (2) Acid-hydrocarbon separation method

Nitration liquid (namely a nitration reaction product containing nitrobenzene) was injected into a sedimentation separation unit 100, so that the nitration liquid was subjected to gravity settling separation by passing through a corrugated plate filler, the intersection angle of the corrugated structure on the corrugated plate filler relative to the horizontal direction was 60°, the separated mixed acids were discharged from an outlet at the bottom, and the separated first oil phase was conveyed into a particle coalescence separation unit 200 from an outlet at the top.

A first oil phase (with an acid amount of 12,000 mg/L) derived from the sedimentation separation unit 100 entered a particle coalescence separation unit 200 at the flow velocity of 6m³/h for subjecting to treatment, wherein a first bed layer 2-2 was formed by filling glass particles with a particle diameter of 0.6-1.2mm, and a second bed layer 2-4 was formed by filling and mixing the polystyrene particles with a particle diameter of 0.3-0.8mm and the ceramic particles in the volume ratio of 1: 2, the ratio of the height of the first bed layer 2-2 to the height of the second bed layer 2-4 was 1: 1, the acid amount in the separated second oil phase was 463 mg/L.

The second oil phase derived from the particle coalescence separation unit 200 entered the first mixer 300, an alkali liquor (sodium hydroxide solution with a concentration of 15wt%) was injected via a second liquid inlet 22, wherein the volume flow ratio of the alkali liquor to the second oil phase was 0.1: 1, the obtained mixed liquor was fed into the first separator 400, wherein the median particle diameter of the hydrocarbon dispersed phase droplets in the mixed solution was 34µm.

The mixed liquor from the first mixer 300 was subjected to demulsification and liquid phase delamination in the first separator 400, wherein the rectifier 4-2 was a perforated flat plate with a pore size of 2mm, a pore center-to-center spacing of 6mm, and a perforation rate of 25%; the first demulsification separation structure 4-3 had a volume specific surface area of 5,000 m²/m³, a porosity of 0.75 and a structure depth of 400mm, and was woven from hydrophilic fibers according to an X-type weaving method; the second demulsification separation structure 4-4 had a volume specific surface area of 5,000 m²/m³, a porosity of 0.75 and a structure depth of 400mm, and was woven from hydrophilic fibers and hydrophobic fibers in a mixing ratio of 1:1 according to an Ω-shaped fiber weaving method; the third demulsification separation structure 4-5 had a structural depth of 400mm, the intersection angle of the corrugated structure on the corrugated plate relative to the horizontal direction was 30°, the materials polypropylene and monel alloy were alternately arranged, a third oil phase was separated from the liquid phase delamination, and the water content in the third oil phase was 381 mg/L.

The third oil phase derived from the first separator 400 entered the second mixer 500, and water was injected via a second liquid inlet 22, wherein the volume flow ratio of water to the third oil phase was 0.2: 1, the obtained mixed liquor was fed into the second separator 600, wherein the median particle diameter of the hydrocarbon dispersed phase droplets in the mixed liquor was 28µm.

The mixed liquor from the second mixer 500 was subjected to demulsification and liquid phase delamination in the second separator 600, washing water was separated from the liquid phase delamination and used for back flushing the particle coalescence separation unit 200, and the purified nitrobenzene was separated from the liquid phase delamination, wherein the water content was 342mg/L, and the nitrophenol content was 5ppm; the salt content in the washing water was 4,251 mg/L.

### Example 2

The acid-hydrocarbon separation system was configured according to Example 1, wherein the specific acid-hydrocarbon separation method was as follows:
Nitration liquid (namely a nitration reaction product containing nitrobenzene) was injected into a sedimentation separation unit 100, so that the nitration liquid was subjected to gravity settling separation by passing through a corrugated plate filler, the intersection angle of the corrugated structure on the corrugated plate filler relative to the horizontal direction was 50°, the separated mixed acids were discharged from an outlet at the bottom, and the separated first oil phase was conveyed into a particle coalescence separation unit 200 from an outlet at the top.

A first oil phase (with an acid amount of 12,000 mg/L) derived from the sedimentation separation unit 100 entered a particle coalescence separation unit 200 at the flow velocity of 6m³/h for subjecting to treatment, wherein a first bed layer 2-2 was formed by filling ceramic particles with a particle diameter of 1.5-3.5mm, and a second bed layer 2-4 was formed by filling and mixing the polypropylene particles with a particle diameter of 0.8-2.0mm and the glass particles in the volume ratio of 1: 1, the ratio of the height of the first bed layer 2-2 to the height of the second bed layer 2-4 was 1: 1, the acid amount in the separated second oil phase was 485 mg/L.

The second oil phase derived from the particle coalescence separation unit 200 entered the first mixer 300, an alkali liquor (sodium hydroxide solution with a concentration of 15wt%) was injected via a second liquid inlet 22, wherein the volume flow ratio of the alkali liquor to the second oil phase was 0.08: 1, the obtained mixed liquor was fed into the first separator 400, wherein the median particle diameter of the hydrocarbon dispersed phase droplets in the mixed solution was 37µm.

The mixed liquor from the first mixer 300 was subjected to demulsification and liquid phase delamination in the first separator 400, wherein the rectifier 4-2 was a perforated flat plate with a pore size of 1mm, a pore center-to-center spacing of 5mm, and a perforation rate of 20%; the first demulsification separation structure 4-3 had a volume specific surface area of 5,000 m²/m³, a porosity of 0.75 and a structure depth of 400mm, and was woven from hydrophilic fibers according to an X-type weaving method; the second demulsification separation structure 4-4 had a volume specific surface area of 5,000 m²/m³, a porosity of 0.75 and a structure depth of 400mm, and was woven from hydrophilic fibers and hydrophobic fibers in a mixing ratio of 1:1.5 according to an Ω-shaped fiber weaving method; the third demulsification separation structure 4-5 had a structural depth of 400mm, the intersection angle of the corrugated structure on the corrugated plate relative to the horizontal direction was 45°, the materials polypropylene and monel alloy were alternately arranged, a third oil phase was separated from the liquid phase delamination, and the water content in the third oil phase was 389 mg/L.

The third oil phase derived from the first separator 400 entered the second mixer 500, and water was injected via a second liquid inlet 22, wherein the volume flow ratio of water to the third oil phase was 0.3: 1, the obtained mixed liquor was fed into the second separator 600, wherein the median particle diameter of the hydrocarbon dispersed phase droplets in the mixed liquor was 33µm.

The mixed liquor from the second mixer 500 was subjected to demulsification and liquid phase delamination in the second separator 600, washing water was separated from the liquid phase delamination and used for back flushing the particle coalescence separation unit 200, and the purified nitrobenzene was separated from the liquid phase delamination, wherein the water content was 346mg/L, and the nitrophenol content was 6ppm; the salt content in the washing water was 4,432mg/L.

### Example 3

The acid-hydrocarbon separation system was configured according to Example 1, wherein the specific acid-hydrocarbon separation method was as follows:
Nitration liquid (namely a nitration reaction product containing nitrobenzene) was injected into a sedimentation separation unit 100, so that the nitration liquid was subjected to gravity settling separation by passing through a corrugated plate filler, the intersection angle of the corrugated structure on the corrugated plate filler relative to the horizontal direction was 70°, the separated mixed acids were discharged from an outlet at the bottom, and the separated first oil phase was conveyed into a particle coalescence separation unit 200 from an outlet at the top.

A first oil phase (with an acid amount of 12,000 mg/L) derived from the sedimentation separation unit 100 entered a particle coalescence separation unit 200 at the flow velocity of 6m³/h for subjecting to treatment, wherein a first bed layer 2-2 was formed by filling ore particles with a particle diameter of 2.5-4.5mm, and a second bed layer 2-4 was formed by filling and mixing the polyurethane particles with a particle diameter of 1.8-3.0mm and the ceramic particles in the volume ratio of 1: 3, the ratio of the height of the first bed layer 2-2 to the height of the second bed layer 2-4 was 1: 1, the acid amount in the separated second oil phase was 498 mg/L.

The second oil phase derived from the particle coalescence separation unit 200 entered the first mixer 300, an alkali liquor (sodium hydroxide solution with a concentration of 15wt%) was injected via a second liquid inlet 22, wherein the volume flow ratio of the alkali liquor to the second oil phase was 0.12: 1, the obtained mixed liquor was fed into the first separator 400, wherein the median particle diameter of the hydrocarbon dispersed phase droplets in the mixed solution was 39µm.

The mixed liquor from the first mixer 300 was subjected to demulsification and liquid phase delamination in the first separator 400, wherein the rectifier 4-2 was a perforated flat plate with a pore size of 3mm, a pore center-to-center spacing of 8mm, and a perforation rate of 40%; the first demulsification separation structure 4-3 had a volume specific surface area of 5,000 m²/m³, a porosity of 0.75 and a structure depth of 400mm, and was woven from hydrophilic fibers according to an X-type weaving method; the second demulsification separation structure 4-4 had a volume specific surface area of 5,000 m²/m³, a porosity of 0.75 and a structure depth of 400mm, and was woven from hydrophilic fibers and hydrophobic fibers in a mixing ratio of 1:2 according to an Ω-shaped fiber weaving method; the third demulsification separation structure 4-5 had a structural depth of 400mm, the intersection angle of the corrugated structure on the corrugated plate relative to the horizontal direction was 60°, the materials polypropylene and monel alloy were alternately arranged, a third oil phase was separated from the liquid phase delamination, and the water content in the third oil phase was 397 mg/L.

The third oil phase derived from the first separator 400 entered the second mixer 500, and water was injected via a second liquid inlet 22, wherein the volume flow ratio of water to the third oil phase was 0.25: 1, the obtained mixed liquor was fed into the second separator 600, wherein the median particle diameter of the hydrocarbon dispersed phase droplets in the mixed liquor was 38µm.

The mixed liquor from the second mixer 500 was subjected to demulsification and liquid phase delamination in the second separator 600, washing water was separated from the liquid phase delamination and used for back flushing the particle coalescence separation unit 200, and the purified nitrobenzene was separated from the liquid phase delamination, wherein the water content was 349mg/L, and the nitrophenol content was 8ppm; the salt content in the washing water was 4,573mg/L.

### Example 4

The acid-hydrocarbon separation system was configured according to Example 1 and implemented with the method according to Example 1, except that the acid-hydrocarbon separation system was further provided with a fiber coalescence unit, wherein a fiber bed layer in the fiber coalescence unit was formed by alternatingly stacking hydrophilic fibers and hydrophobic fibers, an oil phase outlet of the particle coalescence separation unit 200 was connected with a feed inlet of the fiber coalescence unit, an oil phase outlet of the fiber coalescence unit was connected with an oil phase inlet of the first mixer 300, the hydrophilic fibers were Monel alloy fibers, the hydrophobic fibers were Teflon fibers, the volume ratio of the hydrophilic fibers and the hydrophobic fibers was 1:2. Wherein the volumetric flow ratio of the alkali liquor to the second oil phase was 0.06:1.

As a result, the finally separated purified nitrobenzene had a water content of 337mg/L and a nitrophenol content of 3ppm; the salt content in the washing water was 2,158mg/L.

### Example 5

The acid-hydrocarbon separation system was configured according to Example 1 and implemented with the method according to Example 1, except that both the first separator 400 and the second separator 600 were not configured with a second demulsification separation structure 4-4.

As a result, the finally separated purified nitrobenzene had a water content of 556mg/L and a nitrophenol content of 15ppm; the salt content in the washing water was 6,954mg/L.

### Comparative Example 1

The acid-hydrocarbon separation system was configured according to Example 1 and implemented with the method according to Example 1, except that the particle coalescence separation unit 200 was replaced with a fiber filter cartridge separator (purchased from Ontario Shanghai Anci Environmental Protection Technology Co., Ltd., model AFBP-1200), wherein the volume flow ratio of the alkali liquor to the second oil phase (i.e., crude nitrobenzene) in the first mixer 300 was 0.35:1.

As a result, the finally separated purified nitrobenzene had a water content of 396mg/L and a nitrophenol content of 35ppm; the salt content in the washing water was 11,297mg/L.

### Comparative Example 2

The acid-hydrocarbon separation system was configured according to Example 1 and implemented with the method according to Example 1, except that the conveying pipe was not configured in the mixer. Specifically, in the first mixer 300, both the alkali liquor and the second oil phase (i.e., crude nitrobenzene) were injected through a first liquid inlet 12 on the housing; in the second mixer 500, both the water and the third oil phase were injected via a first liquid inlet 12 on the housing, wherein the volume flow ratio of the alkali liquor to the second oil phase in the alkali washing process was 0.3:1.

As a result, the median particle diameter of the hydrocarbon dispersed phase droplets in the mixed liquor obtained in the first mixer 300 was larger than 100µm; the median particle diameter of the hydrocarbon dispersed phase droplets in the mixed liquor obtained in the second mixer 500 is larger than 100µm; the finally separated purified nitrobenzene had a water content of 382mg/L and a nitrophenol content of 42ppm; the salt content in the washing water was 12,219 mg/L.

### Comparative Example 3

The acid-hydrocarbon separation system was configured according to Example 1 and implemented with the method according to Example 1, except that the alkali wash was carried out by using one centrifugal extractor for replacing the first mixer 300 and the first separator 400, and the water washing was performed by using another centrifugal extractor for replacing the second mixer 500 and the second separator 600, wherein the volumetric flow ratio of the alkali liquor to the second oil phase (i.e., crude nitrobenzene) in the alkali washing process was 1:1, and the volumetric flow ratio of water to the third oil phase during the water washing process was 1:1.

As a result, the finally separated purified nitrobenzene had a water content of 849mg/L and a nitrophenol content of 46ppm; the salt content in the washing water was 13,237mg/L.

Table 1 below showed parameter indexes in the material flow of various stages in the aforementioned Examples and Comparative Examples.

**Table 1**

| Example No. | Ex. 1 | Ex. 2 | Ex. 3 | Ex. 4 | Ex. 5 | Comp. Ex. 1 | Com. Ex. 2 | Comp. Ex. 3 |
|---|---|---|---|---|---|---|---|---|
| Acid amount in the first oil phase (mg/L) | 12,000 | 12,000 | 12,000 | 12,000 | 12,000 | 12,000 | 12,000 | 12,000 |
| Acid amount in the second oil phase (mg/L) | 463 | 485 | 498 | 169 | 463 | 1371 | 463 | 463 |
| Median particle diameter of hydrocarbon dispersed phase droplets in the mixed liquor obtained from the first mixer (µm) | 34 | 37 | 39 | 34 | 34 | 38 | >100 | - |
| Water content of the third oil phase separated from the first separator (mg/L) | 381 | 389 | 397 | 384 | 568 | 394 | 391 | - |
| Median particle diameter of hydrocarbon dispersed phase droplets in the mixed liquor obtained from the second mixer (µm) | 28 | 33 | 38 | 28 | 28 | 46 | >100 | - |
| Water content of the purified nitrobenzene (mg/L) | 342 | 346 | 349 | 337 | 556 | 396 | 382 | 849 |
| Nitrophenol content in the purified nitrobenzene (ppm) | 5 | 6 | 8 | 3 | 15 | 27 | 42 | 46 |
| Salt content in washing water (mg/L) | 4,251 | 4,432 | 4,573 | 2,158 | 6,954 | 11,297 | 12,219 | 13,237 |

As can be seen from the results of Table 1, the separation and purification of the nitration reaction product containing nitrobenzene are performed according to the acid-hydrocarbon separation method of the present invention, and can achieve the desirable acid-hydrocarbon separation effects, the content of the nitrophenol in the separated purified nitrobenzene is obviously low, the used amount of alkali liquor is significantly reduced, and the salt content in washing water produced after water washing is obviously low.

### Example 6

The acid-hydrocarbon separation system was configured according to Example 1 for the separation and purification of the nitration reaction product containing nitrotoluene, the specific process was as follows:

Nitration liquid (namely a nitration reaction product containing nitrotoluene) was injected into a sedimentation separation unit 100, so that the nitration liquid was subjected to gravity settling separation by passing through a corrugated plate filler, the separated mixed acids were discharged from an outlet at the bottom, and the separated first oil phase was conveyed into a particle coalescence separation unit 200 from an outlet at the top.

A first oil phase (with an acid amount of 11,868 mg/L) derived from the sedimentation separation unit 100 entered a particle coalescence separation unit 200 at the flow velocity of 6m³/h for subjecting to treatment, the acid amount in the separated second oil phase was 457 mg/L.

The second oil phase derived from the particle coalescence separation unit 200 entered the first mixer 300, an alkali liquor (sodium hydroxide solution with a concentration of 15wt%) was injected via a second liquid inlet 22, wherein the volume flow ratio of the alkali liquor to the second oil phase was 0.15: 1, the obtained mixed liquor was fed into the first separator 400, wherein the median particle diameter of the hydrocarbon dispersed phase droplets in the mixed solution was 35µm.

The mixed liquor from the first mixer 300 was subjected to demulsification and liquid phase delamination in the first separator 400, a third oil phase was separated from the liquid phase delamination, and the water content in the third oil phase was 383 mg/L.

The third oil phase derived from the first separator 400 entered the second mixer 500, and water was injected via a second liquid inlet 22, wherein the volume flow ratio of water to the third oil phase was 0.25: 1, the obtained mixed liquor was fed into the second separator 600, wherein the median particle diameter of the hydrocarbon dispersed phase droplets in the mixed liquor was 30µm.

The mixed liquor from the second mixer 500 was subjected to demulsification and liquid phase delamination in the second separator 600, washing water was separated from the liquid phase delamination and used for back flushing the particle coalescence separation unit 200, and the purified nitrotoluene was separated from the liquid phase delamination, wherein the water content was 341 mg/L, the salt content in the washing water was 4,162 mg/L.

### Comparative Example 4

The acid-hydrocarbon separation system was configured according to Example 6 and implemented with the method according to Example 6, except that the conveying pipe was not configured in the mixers. Specifically, in the first mixer 300, both the alkali liquor and the second oil phase (i.e., crude nitrobenzene) were injected through a first liquid inlet 12 on the housing; in the second mixer 500, both the water and the third oil phase were injected via a first liquid inlet 12 on the housing, wherein the volume flow ratio of the alkali liquor to the second oil phase in the alkali washing process was 0.36:1.

As a result, the median particle diameter of the hydrocarbon dispersed phase droplets in the mixed liquor obtained in the first mixer 300 was larger than 100µm; the median particle diameter of the hydrocarbon dispersed phase droplets in the mixed liquor obtained in the second mixer 500 is larger than 100µm; the finally separated purified nitrotoluene had a water content of 382 mg/L, the salt content in the washing water was 11,579 mg/L.

As can be seen from a comparison of Example 6 with Comparative Example 4, the separation and purification of the nitration reaction product containing nitrotoluene performed according to the acid-hydrocarbon separation method of the present invention can produce the desired acid-hydrocarbon separation effect, the used amount of alkali liquor is obviously reduced, and the salt content in washing water produced after water washing is obviously low.

### Example 7

The acid-hydrocarbon separation system was configured according to Example 1 for the separation and purification of the nitration reaction product containing nitrochlorobenzene, the specific process was as follows:
Nitration liquid (namely a nitration reaction product containing nitrochlorobenzene) was injected into a sedimentation separation unit 100, so that the nitration liquid was subjected to gravity settling separation by passing through a corrugated plate filler, the separated mixed acids were discharged from an outlet at the bottom, and the separated first oil phase was conveyed into a particle coalescence separation unit 200 from an outlet at the top.

A first oil phase (with an acid amount of 11,594 mg/L) derived from the sedimentation separation unit 100 entered a particle coalescence separation unit 200 at the flow velocity of 6m³/h for subjecting to treatment, the acid amount in the separated second oil phase was 453 mg/L.

The second oil phase derived from the particle coalescence separation unit 200 entered the first mixer 300, an alkali liquor (sodium hydroxide solution with a concentration of 15wt%) was injected via a second liquid inlet 22, wherein the volume flow ratio of the alkali liquor to the second oil phase was 0.12: 1, the obtained mixed liquor was fed into the first separator 400, wherein the median particle diameter of the hydrocarbon dispersed phase droplets in the mixed solution was 33µm.

The mixed liquor from the first mixer 300 was subjected to demulsification and liquid phase delamination in the first separator 400, a third oil phase was separated from the liquid phase delamination, and the water content in the third oil phase was 379 mg/L.

The third oil phase derived from the first separator 400 entered the second mixer 500, and water was injected via a second liquid inlet 22, wherein the volume flow ratio of water to the third oil phase was 0.23: 1, the obtained mixed liquor was fed into the second separator 600, wherein the median particle diameter of the hydrocarbon dispersed phase droplets in the mixed liquor was 31 µm.

The mixed liquor from the second mixer 500 was subjected to demulsification and liquid phase delamination in the second separator 600, washing water was separated from the liquid phase delamination and used for back flushing the particle coalescence separation unit 200, and the purified nitrochlorobenzene was separated from the liquid phase delamination, wherein the water content was 343 mg/L, the salt content in the washing water was 4,129 mg/L.

### Comparative Example 5

The acid-hydrocarbon separation system was configured according to Example 7 and implemented with the method according to Example 7, except that the conveying pipe was not configured in the mixers. Specifically, in the first mixer 300, both the alkali liquor and the second oil phase (i.e., crude nitrobenzene) were injected through a first liquid inlet 12 on the housing; in the second mixer 500, both the water and the third oil phase were injected via a first liquid inlet 12 on the housing, wherein the volume flow ratio of the alkali liquor to the second oil phase in the alkali washing process was 0.35:1.

As a result, the median particle diameter of the hydrocarbon dispersed phase droplets in the mixed liquor obtained in the first mixer 300 was larger than 100µm; the median particle diameter of the hydrocarbon dispersed phase droplets in the mixed liquor obtained in the second mixer 500 is larger than 100µm; the finally separated purified nitrotoluene had a water content of 387 mg/L, the salt content in the washing water was 11,357 mg/L.

As can be seen from a comparison of Example 7 with Comparative Example 5, the separation and purification of the nitration reaction product containing nitrochlorobenzene performed according to the acid-hydrocarbon separation method of the present invention can produce the desired acid-hydrocarbon separation effect, the used amount of alkali liquor is obviously reduced, and the salt content in washing water produced after water washing is obviously low.

The above content describes in detail the preferred embodiments of the present invention, but the present invention is not limited thereto. A variety of simple modifications can be made in regard to the technical solutions of the present invention within the scope of the technical concept of the present invention, including a combination of individual technical features in any other suitable manner, such simple modifications and combinations thereof shall also be regarded as the content disclosed by the present invention, each of them falls into the protection scope of the present invention.

## Claims

1. A mixer, is **characterized in that** the mixer comprises:
a housing (1), wherein a mixing cavity (11) is provided inside the housing (1), a first liquid inlet (12) through which a raw material liquid enters the mixing cavity (11) is provided on the head end of the housing (1), and a liquid mixture outlet (13) through which a liquid mixture flows out of the mixing cavity (11) is provided on the tail end of the housing (1); and
a conveying pipe (2), the conveying pipe (2) running through the mixing cavity (11) via the head end of the housing (1) and extending to the tail end of the housing (1), wherein a first port of the conveying pipe (2) located at the head end extends out of the housing (1) and forms a second liquid inlet (22), a second port of the conveying pipe (2) located at the tail end is closed, the part of the conveying pipe (2) located in the mixing cavity (11) forms a spraying section (21), a plurality of spraying holes (23) distributed at intervals and configured to spray to the mixing cavity (11) a dispersed phase inputted through the second liquid inlet (22) are provided in the pipe wall of the spraying section (21), and a first helical blade (24) and a second helical blade (25) which are distributed at an interval in the axial direction of the spraying section (21) are provided on the periphery of the spraying section (21), with the helical direction of the first helical blade (24) being opposite to the helical direction of the second helical blade (25).

2. The mixer according to claim 1, wherein the mixing cavity (11) comprises a first variable diameter zone (14), a buffer zone (15) and a second variable diameter zone (16) distributed in sequence along the direction from the first liquid inlet (12) to the liquid mixture outlet (13), the first helical blade (24) and the second helical blade (25) are located in a first variable diameter zone (14) and a second variable diameter zone (16) respectively;
and/or, the housing (1) is cylindric, an axial direction of the first liquid inlet (12) is perpendicular to an axial direction of the housing (1), and an axial direction of the liquid mixture outlet (13) is the same as an axial direction of the housing (1).

3. The mixer according to claim 2, wherein the first variable diameter zone (14) comprises a first mixing section (141), a first expansion section (142) and a first tapered section (143) sequentially distributed along an axial direction of the spraying section (21); wherein the first mixing section (141) is in communication with the first liquid inlet (12), the first expansion section (142) is in communication with the buffer zone (15);
the diameter of the first tapered section (143) gradually decreases along a direction from the first liquid inlet (12) to the liquid mixture outlet (13), the minimum diameter of the first tapered section (143) is the same as the diameter of the buffer zone (15), the maximum diameter of the first tapered section (143) is the same as the diameter of the first expansion section (142), and the diameter of the first expansion section (142) is larger than the diameter of the first mixing section (141).

4. The mixer according to claim 3, wherein a first radial spacing is provided between a wall surface of the first mixing section (141) and an outer edge of the first helical blade (24), the minimum radial spacing between the wall surface of the first tapered section (143) and the outer edge of the first helical blade (24) is a second radial spacing, and a third radial spacing is provided between an outer wall surface of the spraying section (21) and the wall surface of the first mixing section (141), the first radial spacing and the second radial spacing are 1/8 - 1/4 of the third radial spacing.

5. The mixer according to claim 3, wherein a wall surface of the first expansion section (142) is provided with a first spiral linear repoussage (144) encircling the first helical blade (24), the helical direction of the first spiral linear repoussage (144) is opposite to the helical direction of the first helical blade (24).

6. The mixer according to claim 4, wherein an axial length of the second variable diameter zone (16) is smaller than an axial length of the first variable diameter zone (14);
the second variable diameter zone (16) comprises a second mixing section (161), a second expansion section (162) and a second tapered section (163) distributed in sequence along an axial direction of the spraying section (21), the second mixing section (161) is in communication with the buffer zone (15), the second tapered section (163) is in communication with the liquid mixture outlet (13);
the diameter of the second tapered section (163) gradually decreases along a direction from the second liquid inlet (22) to the liquid mixture outlet (13), the minimum diameter of the second tapered section (163) is the same as the diameter of the liquid mixture outlet (13), the maximum diameter of the second tapered section (163) is the same as the diameter of the second expansion section (162), the diameter of the second expansion section (162) is larger than the diameter of the second mixing section (161), the diameter of the second mixing section (161) is equal to the diameter of the buffer zone (15).

7. The mixer according to claim 6, wherein a fourth radial spacing is provided between a wall surface of the second mixing section (161) and an outer edge of the second helical blade (25), a minimum radial spacing between a wall surface of the second tapered section (163) and an outer edge of the second helical blade (25) is a fifth radial spacing, a sixth radial spacing is provided between an outer wall surface of the spraying section (21) and a wall surface of the second mixing section (161), the fourth radial spacing and the fifth radial spacing are 1/8 - 1/4 of the sixth radial spacing.

8. The mixer according to claim 6, wherein a wall surface of the second expansion section (162) is provided with a second spiral linear repoussage (164) encircling the second helical blade (25), the helical direction of the second spiral linear repoussage (164) is opposite to the helical direction of the second helical blade (25).

9. The mixer according to claim 6, wherein all the spraying holes (23) are distributed in multiple rows and columns on the spraying section (21), and the number of the spraying holes (23) in the first variable diameter zone (14) is greater than the number of the spraying holes (23) in the second variable diameter zone (16);
in the axial direction of the spraying section (21), the spacing between two neighboring spraying holes (23) in the buffer zone (15) is smaller than the spacing between two neighboring spraying holes (23) in the second variable diameter zone (16).

10. The mixer according to claim 9, wherein the pore diameter of the spraying holes (23) gradually decreases from their liquid inlet end to their liquid outlet end, the pore walls of the spraying holes (23) are provided with a third spiral linear repoussage (26) distributed around an axis of the spraying holes (23), the spraying holes (23) extend obliquely from their liquid inlet end to their liquid outlet end towards the head end of the housing (1).

11. An acid-hydrocarbon separation system, is **characterized in that** the acid-hydrocarbon separation system comprises: a sedimentation separation unit (100), a coalescence separation unit, an alkali washing unit and a water washing unit connected in sequence, the alkali washing unit comprises a first mixer (300) and a first separator (400) connected in sequence, wherein the first mixer is the mixer according to any one of claims 1-10.

12. The acid-hydrocarbon separation system according to claim 11, wherein the sedimentation separation unit (100) is a gravity settling separator.

13. The acid-hydrocarbon separation system according to claim 11 or 12, wherein the coalescence separation unit comprises one or more particle coalescence separation units (200), an inner cavity of the particle coalescence separation units (200) is provided with a particle bed layer formed by media particles, which are hydrophilic particles, or a combination of hydrophilic particles and hydrophobic particles.

14. The acid-hydrocarbon separation system according to claim 13, wherein the coalescence separation unit further comprises one or more fiber coalescence separation units, the fiber coalescence separation unit and the particle coalescence separation unit (200) are connected in series with each other, an inner cavity of the fiber coalescence separation unit is provided with a fiber bed layer formed by fiber media comprising hydrophilic fibers and hydrophobic fibers.

15. The acid-hydrocarbon separation system according to claim 14, wherein the fiber bed layer is formed by stacking a plurality of fiber layers, each of the fiber layers is woven from hydrophilic fibers and hydrophobic fibers.

16. The acid-hydrocarbon separation system according to any one of claims 11-15, wherein the first separator (400) comprises: a second housing; and a second liquid distributor (4-1), a rectifier (4-2), a first demulsification separation structure (4-3), a second demulsification separation structure (4-4) and a third demulsification separation structure (4-5) sequentially arranged in an inner cavity of the second housing along a material flow direction, wherein the first demulsification separation structure (4-3) is a layer of hydrophilic fibers, the second demulsification separation structure (4-4) is an interlaced layer of lipophilic fibers and hydrophilic fibers, and the third demulsification separation structure (4-5) is a corrugated plate.

17. The acid-hydrocarbon separation system according to claim 16, wherein the corrugated plate in the first separator (400) is formed by alternating stacking of oleophilic materials and hydrophilic materials;
preferably, the angle between the corrugation structure on the corrugated plate and the horizontal direction is 30°-60°;
preferably, both the peaks and valleys of the corrugated plate are provided with holes.

18. The acid-hydrocarbon separation system according to any one of claims 11-17, wherein the water washing unit comprises a second mixer (500) and a second separator (600) connected with each other;
preferably, the structure of the second mixer (500) is the same as that of the first mixer (300);
preferably, the structure of the second separator (600) is the same as that of the first separator (400).

19. An acid-hydrocarbon separation method, is **characterized in that** the method comprises the following steps:
(1) Subjecting an acid-hydrocarbon mixture to sedimentation separation to obtain a first oil phase;
(2) Subjecting the first oil phase to coalescence separation such that the acid content of the separated second oil phase is 5% or less, preferably 0.1-4.2% of the acid content of the first oil phase;
(3) Performing a first mixing of the second oil phase with an alkali liquor, and subjecting the obtained mixed liquor to a first enhanced separation to obtain a third oil phase;
(4) Subjecting the third oil phase to water washing.

20. The method according to claim 19, wherein the coalescence separation process in step (2) comprises:
contacting the first oil phase with media particles for carrying out a first coalescence separation, wherein the media particles are hydrophilic particles, or a combination of hydrophilic particles and hydrophobic particles;
optionally, the oil phase obtained from the first coalescence separation contacts with a fiber media for performing a second coalescence separation, wherein the fiber media comprises hydrophilic fibers and hydrophobic fibers.

21. The method according to claim 20, wherein the media particles have a particle diameter within the range of 0.2-5mm.

22. The method according to claim 20 or 21, wherein the hydrophilic particles are at least one selected from the group consisting of ore particles, ceramic particles and glass particles; and/or
the hydrophobic particles are at least one selected from the group consisting of polypropylene particles, polystyrene particles, and polyurethane particles.

23. The method according to any one of claims 20-22, wherein the second coalescence separation process comprises: passing the oil phase obtained from the first coalescence separation from top to bottom through a fiber bed layer formed of the fiber media;
preferably, the fiber bed layer is formed by stacking a plurality of fiber layers, each of the fiber layers is woven from hydrophilic fibers and hydrophobic fibers.

24. The method according to claim 19, wherein the first mixing process in step (3) makes the median particle diameter of the hydrocarbon dispersed phase droplets in the mixed liquor obtained after mixing is 100µm or less, preferably 40µm or less.

25. The method according to claim 19, wherein the first enhanced separation process in step (3) causes that the separated third oil phase has a water content being 600mg/L or less, preferably 400mg/L or less.

26. The method according to claim 19, 24 or 25, wherein the volumetric flow ratio of the second oil phase to the alkali liquor in step (3) is 1: (0.05-0.2).

27. The method according to claim 19, wherein the water washing process in step (4) comprises: performing a second mixing on the third oil phase with water, and subjecting the obtained mixed liquor to a second enhanced separation.

28. The method according to claim 27, wherein the second mixing process makes the median particle diameter of the hydrocarbon dispersed phase droplets in the mixed liquor obtained after mixing is 100µm or less, preferably 40µm or less.

29. The method according to claim 27, wherein the second enhanced separation process causes that the separated purified hydrocarbon has a water content being 600mg/L or less, preferably 400mg/L or less.

30. The method according to claim 19, 27, 28 or 29, wherein the volumetric flow ratio of the third oil phase to water is 1: (0.1-0.6).

31. The method according to any one of claims 19-30, wherein the acid in the acid-hydrocarbon mixture is at least one selected from the group consisting of hydrochloric acid, sulfuric acid, nitric acid, phosphoric acid, hydrofluoric acid and hydrobromic acid; and/or
the hydrocarbon in the acid-hydrocarbon mixture is aromatic hydrocarbon, preferably at least one selected from the group consisting of nitroaromatic hydrocarbon, halogenated aromatic hydrocarbon, and nitro-halogenated aromatic hydrocarbon.
